# EUROPEAN PATENT SPECIFICATION

(11) **EP 1 810 705 B1**
(45) Date of publication and mention of the grant of the patent: **19.05.2010**
(21) Application number: 07000985.7
(22) Date of filing: 18.01.2007
(51) Int. Cl.: A61M 1/16

(54) **Oxygenator**
Oxygenator
Oxygénateur

(30) Priority: 19.01.2006 JP 2006011702
(43) Date of publication of application: 25.07.2007
(73) Proprietor: TERUMO KABUSHIKI KAISHA, Tokyo 151-0072 (JP)
(72) Inventor: Mitsuaki, Ogihara, Fujinomiya-shi Shizuoka 418-0015 (JP)
(74) Representative: Casalonga, Axel

(56) References cited:
- EP-A- 1 618 906
- WO-A-00/06357
- WO-A-97/16213
- JP-A- 07 328 114

## Description

The present invention relates to oxygenators.

WO 00/06357 discloses an oxygenator comprising a bundle of hollow fiber membranes. The oxygenator comprises a housing of substantially rectangular shape having a blood outlet on an outer surface; gas exchange layers and heat exchange layers, which are intermittently wrapped around a blood inlet extending axially and centrally through the housing and stacked to fill the housing; and furthermore a filter of single or multiple filament and a screen for supporting the filter, which are arranged in contact with an essentially plane downstream surface of an outer gas exchange layer and close to the blood outlet. The bundle is internally structured so as to comprise plural gas exchange layers constituted of plural hollow fibers arranged mutually parallel in layers formed using interweaving cross-strands and plural heat transfer layers constituted of plural hollow fibers arranged mutually parallel in layers formed using interweaving cross-Strands. The gas exchange layers are provided as sheets and the heat transfer layers are provided as sheets. Sheets of gas exchange layers are "wrapped" cylindrically around the blood oxygenation chamber, a sheet of heat transfer layer is wrapped therearound, and plural further gas exchange layers intermittently with heat transfer layers are wrapped cylindrically. In operation, after travelling through the blood inlet, blood flows radially outward within the oxygenation chamber and thereby passes through several gas exchange layers and also through several heat transfer layers and finally through the filter to the main blood outlet. -- JP 07 328 114 discloses an oxygen applicator for use in extracorporeal circulation . The oxygen applicator has a generally vertical cylindrical form and comprises a basically cylindrically shaped oxygen applying section of the external perfusion type made up of a hollow cylindrical element arid an artery filter provided at an internal part of the oxygen applying section. A blood inflow port and an oxygen outflow port are provided at a lower end of the oxygen applying section, and a blood outflow port, an oxygen inflow port, an air reservoir arranged above the artery filter and a bubble removal port in communication with the air reservoir are provided at the upper end of the oxygen applying section. Conventionally, there are further known oxygenators constructed to perform gas exchange by use of a multiplicity of hollow fiber membranes (e.g. U.S. Pat. No. 6,503,451).

This oxygenator includes a housing, a hollow fiber membrane bundle received in the housing, blood-inlet and blood-outlet ports, and gas-inlet and gas-outlet ports so that gas exchange, i.e. oxygenation and decarboxylation, is performed between blood and gas through the hollow fiber membranes.

In the meanwhile, in the oxygenator constructed like this, bubbles possibly exist in the blood coming through the blood inlet port. In such a case, bubbles are preferably removed by the hollow fiber membrane bundle.

However, the hollow fiber membrane bundle is designed to attempt to efficiently effect gas exchange, i.e. it in nature is not intended to remove bubbles. Thus, there is a problem that bubbles are not fully removed by the hollow fiber membrane bundle with a result that bubbles remaining in the blood go out of the blood outlet port and are carried to the downstream of the oxygenator. For this reason, it is a practice to provide a bubble-removing arterial filter on an arterial line between the oxygenator and the patient.

Therefore, it is an object of the present invention to provide an oxygenator capable of preventing the bubbles in blood from going out of the blood outlet by providing the oxygenators with a function to catch bubbles.
The aforementioned object is solved by providing an oxygenator according to claim 1. As claimed, an oxygenator comprises:
a housing substantially in a rectangular parallelepiped form;
a hollow fiber membrane bundle received in the housing and formed by integrating together a multiplicity of hollow fiber membranes serving for gas exchange, wherein each fiber membrane comprises a lumen structuring a gas passage;
a gas inlet provided at a gas flow upstream portion of the fiber membrane bundle and a gas outlet provided at a gas flow downstream portion of the fiber membrane bundle, so as to provide a gas passage through the lumens of the hollow fiber membranes from the gas flow upstream portion to the gas flow down stream portion;
a blood-inlet portion extending at a blood flow upstream surface of the fiber membrane bundle and a blood-outlet portion extending at a blood flow downstream surface of the fiber membrane bundle, so as to provide a blood passage through the fiber membrane bundle from said blood flow upstream surface to the blood flow downstream surface, wherein the blood passage is formed exterior of the hollow fiber membranes; and
a filter member, serving for catching bubbles, being provided on the blood flow downstream side of the hollow fiber membrane bundle.
According to the invention, the hollow fiber membrane bundle as a whole has a substantially rectangular parallelepiped form. Further according to the invention, the filter member is placed such that one of its surfaces is in contact with said blood flow downstream surface of the fiber membrane bundle so that the filter member covers nearly all of the blood flow downstream surface of the fiber membrane bundle.

According to the present invention, by the provision of a filter member serving to catch bubbles, the bubbles in blood can be positively caught, thus preventing bubbles from exiting through the blood outlet.

Fig. 1 is a perspective view showing an embodiment of an oxygenator according to the present invention;

Fig. 2 is a sectional view (sectional side view) taken along the line X-X in Fig. 1;

Fig. 3 is a cross-sectional view (sectional top view) of an oxygenating part of the oxygenator shown in Fig. 1; and

Fig. 4 is an enlarged sectional view of a lower right region (fixing region of a hollow fiber membrane bundle and a filter member) in Fig. 2.

Based on the preferred embodiment shown in the drawings, an oxygenator of the present invention will now be described in detail.

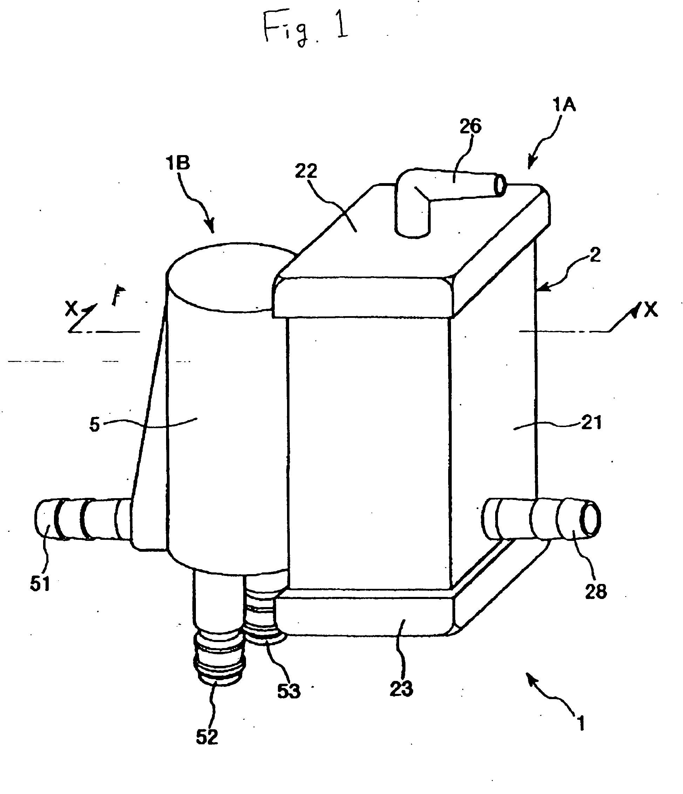

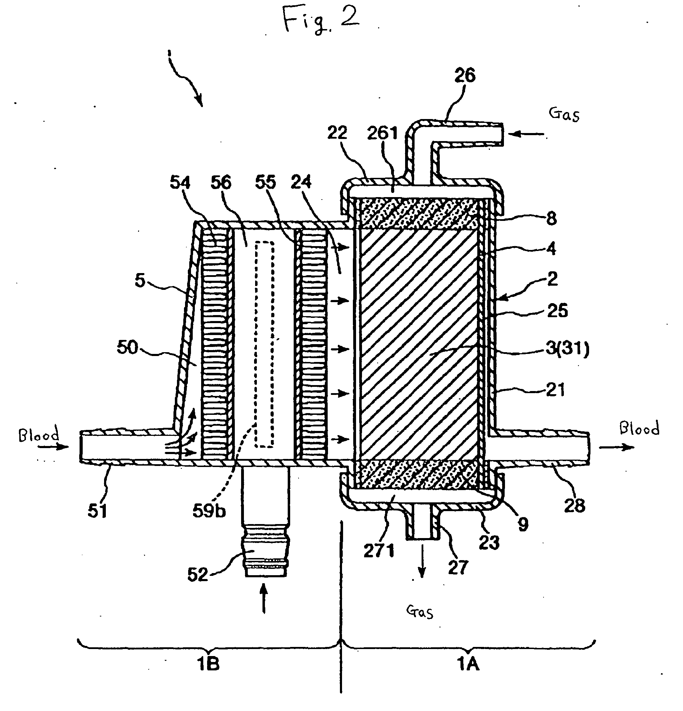

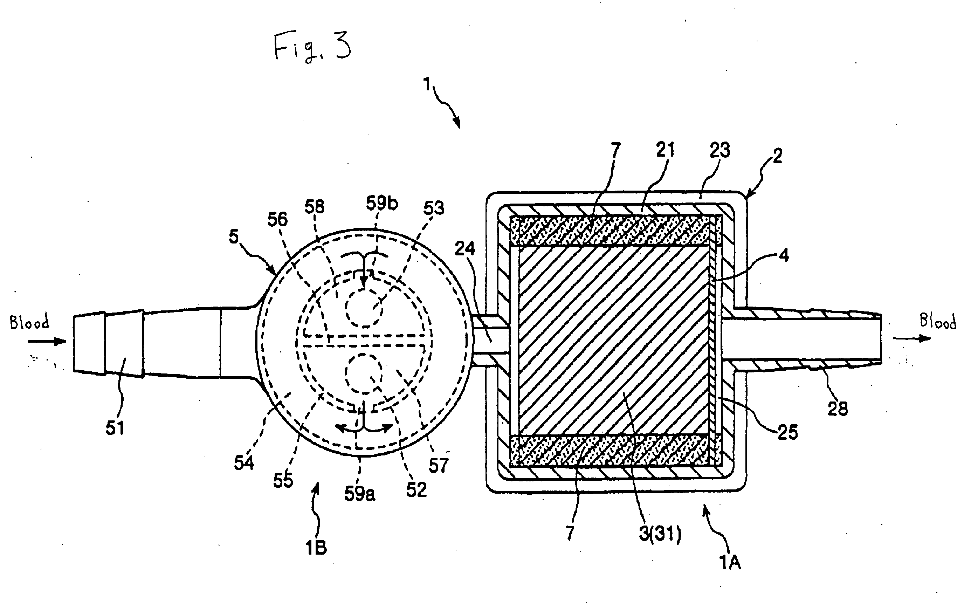

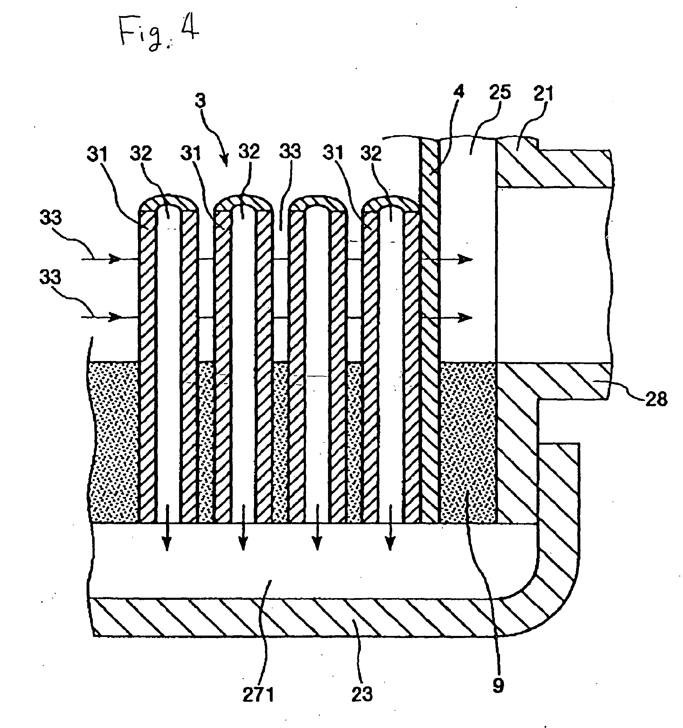

Fig. 1 is a perspective view showing an embodiment of an oxygenator according to the invention, Fig. 2 is a sectional view (sectional side view) taken along line X-X in Fig. 1, Fig. 3 is a cross-sectional view (sectional top view) of an oxygenating part of the oxygenator shown in Fig. 1, and Fig. 4 is an enlarged sectional view of a lower right region (fixing region of a hollow fiber membrane bundle and a filter member) in Fig. 2. Note that, in Figs. 1 and 2, the upper side is referred to resp. taken as "upper" or "above", the lower side is taken as "lower" or "below", the left side is taken as "blood inlet side" or "upstream side", the right side is taken as "blood outlet side" or "downstream side".

An oxygenator 1, in the illustrated embodiment, is a heat exchanger-equipped oxygenator that includes an oxygenating part 1A to perform gas exchange on blood and a heat exchanging part (heat exchanger) 1B to perform heat exchange on blood. This is to be set up on a blood extracorporeal circulation circuit, for example.

The oxygenator 1 has a housing 2 located on the side of the oxygenating part 1A, and a heat exchanger housing 5 located on the side of the heat exchanger 1B. Those are united or integrated in one body. At first, the oxygenating part 1A is explained.

The housing 2 is made up with a cylindrical housing body 21 quadrilateral (rectangle or square) in cross section (hereinafter, referred to as a "rectangularly cylindrical housing body"), a first header (upper lid) 22 in a dish form closing the upper opening of the rectangularly cylindrical housing body 21, and a second header (lower lid) 23 in a dish form closing the lower opening of the rectangularly cylindrical housing body 21.

The rectangularly cylindrical housing body 21, the first header 22 and the second header 23 are each formed of a resin material, e.g. polyolefin such as polyethylene or polypropylene, an ester resin (e.g. polyester such as polyethylene terephthalate or polybutylene terephthalate), a styrene resin (e.g. polystyrene, MS resin or MBS resin) or polycarbonate, a ceramics material in various kind or a metal material. The first and second headers 22, 23 are secured liquid-tight to the rectangularly cylindrical housing body 21 by joining due to fusion or an adhesive.

The rectangularly cylindrical housing body 21 is formed with a tubular blood outlet port 28 projecting in the lower region of the blood outlet side thereof. A tubular gas inlet port 26 is formed projecting from the upper surface of the first header 22. A tubular gas outlet port 27 is formed projecting from the lower surface of the second header 23. The gas inlet port 26 has an intermediate portion bent nearly rectangular and a tip portion directed parallel with the blood outlet port 28.

The housing 2 receives therein a hollow fiber membrane bundle 3 formed by integrating a multiplicity of hollow fiber membranes 31 serving for gas exchange, a filter member 4 serving for catching bubbles.

As shown in Fig. 4, almost all the hollow fiber membranes 31 forming the hollow fiber membrane bundle 3 are arranged nearly parallel with one another. In this case, the hollow fibermembranes 31 are arranged vertical in the lengthwise thereof.

Incidentally, the arrangement pattern, direction, etc. of the hollow fiber membranes 31 in the hollow fiber membrane bundle 3 are not limited to those mentioned but may be, say, in a structure that the hollow fiber membranes 31 are arranged horizontal, a structure that has points at which the hollow fiber membranes 31 obliquely intersect with one another (intersections), a structure that all or part of the hollow fiber membranes 31 are arranged curved, or a structure that all or part of the hollow fiber membranes 31 are arranged corrugated, helical, spiral or annular.

The hollow fiber membranes 31 have opposite ends (upper and lower ends) fixed to the inner surfaces of the rectangularly cylindrical housing body 21 by means of partitioning walls 8, 9 (see Fig. 2). The partitioning walls 8, 9 are formed of a potting material, e.g. polyurethane or silicone rubber.

Meanwhile, the hollow fiber membrane bundle 3 has widthwise opposite ends respectively fixed (secured) to the inner surfaces of the rectangularly cylindrical housing body 21 through setting member 7 (see Fig. 3). The setting member 7 is formed similar in material (potting material) to the partitioning wall 8, 9 or of another material.

A gas inlet chamber 261 is defined by the first header 22 and the partitioning wall 8. The hollow fiber membranes 31 have respective upper openings released to and communicating with the gas inlet chamber 261. Meanwhile, a gas outlet chamber 271 is defined by the second header 23 and the partitioning wall 9. The hollow fiber membranes 31 have respective lower openings released to and communicating with the gas outlet chamber 271. The hollow fiber membrane 31 has a lumen structuring a gas passage 32 through which gas is to flow. The gas inlet port 26 and the gas inlet chamber 261 constitute a gas inlet located in the gas passage 32 at a gas upstream portion thereof while the gas outlet port 27 and the gas outlet chamber 271 constitute a gas outlet located in the gas passage 32 at a gas downstream portion thereof.

The hollow fiber membrane bundle 3 is charged nearly fully in the rectangularly cylindrical housing body 21 so that the hollow fiber membrane bundle 3 wholly resp. as a whole takes a rectangular parallelepiped form. Due to this, high charge efficiency by the hollow fiber membranes 31 is available (with less dead space) in the rectangularly cylindrical housing body 21 similar in form, which contributes to the size reduction and performance improvement of the oxygenating part 1A.

The hollow fiber membranes 31 are exposed between the partitioning walls 8, 9, within the housing 2. A blood passage 33 is formed exterior of the hollow fiber membranes 31 i.e. at gaps between the hollow fiber membranes 31, allowing blood to flow from left to right in Fig. 2.

A blood inlet aperture (blood inlet space) 24 is formed as a blood inlet in a stripe or slit form extending vertically (nearly parallel with the arrangement of the hollow fiber membranes 31), in the blood passage 33 at an upstream portion thereof (closer to an upstream surface of the hollow fiber membrane bundle 3), i.e. in a connection of between the rectangularly cylindrical housing body 21 and the heat exchanger housing 5. The housing 2 has an interior in communication with an interior of the heat exchanger housing 5, through the blood inlet aperture 24. This structure allows for efficient transfer of blood from the heat exchanging part 1B to the oxygenating part 1A.

The blood inlet aperture 24 preferably has a length (vertical length) nearly equal to (see Fig. 2) or somewhat shorter (by 70% or more of the effective length) than the effective length of the hollow fiber membrane 31 (length of between a partitioning wall 8 lower face and a partitioning wall 9 upper face). This allows for efficient transfer of blood from the heat exchanging part 1B to the oxygenating part 1A and for gas exchange of blood in the blood passage 33.

Meanwhile, in the blood passage 33 at least in an upstream portion thereof (closer to the blood inlet aperture 24), the flow of blood is in a direction nearly orthogonal to the lengthwise direction of the hollow fiber membranes 31. This allows for efficient gas exchange on the blood flowing the blood passage 33.

In the blood passage 33 at a downstream portion thereof (closer to the downstream surface of the hollow fiber membrane bundle 3), a gap is formed between a filter member 4, referred later, and an inner surface of the rectangularly cylindrical housing body 21. The gap forms a blood outlet aperture (blood outlet space) 25. A blood outlet is constituted by the blood outlet aperture 25 and the blood outlet port 28 communicating with the blood outlet aperture 25. By having the blood outlet aperture 25, the blood outlet is secured with a space where the blood transmitted through the filter member 4 is to flow toward the blood outlet port 28, thus discharging the blood smoothly.

Between the blood inlet aperture 24 and the blood outlet aperture 25, there exist the hollow fiber membrane bundle 3, the filter member 4, and the blood passage 33.

The hollow fiber membrane 31 uses a porous gas-exchange membrane, for example. The porous hollow fiber membrane can use an inner diameter of approximately 100 - 1000 µm, a wall thickness of approximately 5 - 200 µm or preferably 10 - 100 µm, a porosity of approximately 20 - 80% or preferably approximately 30 - 60%, a pore size of approximately 0.01 - 5 µm or preferably approximately 0.01 - 1 µm.

Meanwhile, the hollow fiber membrane 31 uses a hydrophobic polymer material, e.g. polypropylene, polyethylene, polysulfone, polyacrylonitrile, polytetrafluoroethylene or polymethyl pentane. Polyolefin resin is preferred, and polypropylene is more preferred. Pores are preferably formed in a wall by stretching or solid-liquid phase separation.

The hollow fiber membranes 31 of the hollow fiber membrane bundle 3 have a length (effective length) that is not especially limited but is preferably approximately 30 - 150 mm, more preferably approximately 50 - 100 mm.

The hollow fiber membrane bundle 3 has a thickness (horizontal length in Fig. 2) that is not especially limited but is preferably approximately 10 - 100 mm, more preferably approximately 20 - 80 mm.

The hollow fiber membrane bundle 3 has a width (vertical length in Fig. 3) that is not especially limited but is preferably approximately 10 - 100 mm, more preferably approximately 20 - 80 mm.

The filter member 4 is provided in a position downstream of the hollow fiber membrane bundle 3 (closer to the blood outlet), which serves to catch the bubbles in blood flowing the blood passage 33. The filter member 4 is formed by a flat sheet member nearly in a rectangular form (hereinafter, also referred merely to as a "sheet"). The filter member 4 is fixed in the housing 2 by being secured at its edges (four sides) through the partitioning walls 8, 9 and the respective setting members 7.

The filter member 4 is provided by placing its one surface in contact with the downstream surface (closer to the blood outlet portion) of the hollow fiber membrane bundle 3, thus covering nearly all the surface. By thus providing the filter member 4, the filter member 4 has an increased effective area, thus making it possible to fully exhibit the capability of catching bubbles. Meanwhile, by increasing the effective area of the filter member 4, even if clogging (e.g. adhesion of blood aggregates) occurs in a part of the filter member 4, it can be prevented (suppressed) wholly from obstructing the blood flow.

The filter member 4 may be, say, in a mesh form or of, a woven fabric, a non-woven fabric or a combination thereof. Of these, mesh form is preferred, and particularly, a screen filter is preferred. This makes it possible to catch bubbles more positively and to pass blood easily.

In the case the filter member 4 is in a mesh form, the mesh size is not limited but usually preferably 80 µm or smaller, more preferably approximately 15 - 60 µm, further preferably 20 - 45 µm. This makes it possible to catch comparatively fine bubbles without increasing the passage resistance to blood, thus providing a high catch efficiency of bubbles (removal capability).

The filter member 4 is made of a material, e.g. polyolefin such as polyamide, polyethylene or polypropylene, polyester such as polyethylene terephthalate, or polybutylene terephthalate, nylon, cellulose, polyurethane, or an aramid fiber. Particularly, it is preferable to use polyethylene terephthalate, polyethylene or polyurethane in respect of the excellent resistance to blood clotting and the capability to be less clogged.

Meanwhile, the filter member 4 preferably has hydrophilicity. Namely, the filter member 4 preferably is made itself of a hydrophilic material or have been subjected to a hydrophilizing processing (e.g. plasma processing). This makes it easy to remove bubbles at a priming of the oxygenator 1. Besides, when the blood mixed with bubbles passes, the bubbles are difficult to pass through, thus improving the bubble removal capability at the filter member 4 and preventing positively the bubbles from going out of the blood outlet port 28.

The filter member 4 may use one sheet (particularly, a mesh form like a screen filter) or two or more thereof.

Between the filter member 4 and the housing 2, a gap, i.e. blood outlet aperture 25, is formed (see Figs. 2 and 3) . This can suppress the filter member 4 from going into direct (close) contact with the inner surface of the housing 2. The blood that has passed through the filter member 4 is allowed to easily flow down the blood outlet aperture 25, and then to the blood outlet port 28 smoothly.

The filter member 4 is rectangular (or square) in plane in the illustrated embodiment. However, the plan form of the filter member 4 is not limited to that but may be trapezoidal, parallelogram, elliptic or oval, for example.

By arranging the filter member 4 thus structured, even when bubbles exist in the blood flowing through the blood passage 33, such bubbles can be caught thereby preventing the bubbles from going out of the blood outlet port 28. This eliminates the necessity of an arterial filter conventionally provided on the arterial line.

The bubbles, caught by the filter member 4, are once stored on the upstream side of the filter member 4 where the bubbles go into a lumen (gas passage 32) of the hollow fiber membrane 31 through a multiplicity of fine pores formed in a wall of the hollow fiber membrane 31 arranged nearby the same. The bubbles are discharged at the gas outlet port 27, together with the gas flowing through the gas passage 32. This eliminates the necessity of an arterial filter, hence reducing the priming time at a start in using the oxygenator and preventing the adverse effect caused due to the bubbles staying in the blood passage 33 (e.g. lowered gas-exchange capability of the hollow fiber membrane bundle 3).

Now description is made of the heat exchanging part (heat exchanger) 1B. The heat exchanger 1B has a heat exchanger housing 5. The heat exchanger housing 5 is nearly in a cylindrical form having upper and lower ends closed. The heat exchanger housing 5 is formed therein with a blood chamber 50. The heat exchanger housing 5 is formed with a tubular heating medium inlet port 52 and a heating medium outlet port 53, projecting at a lower end (lower surface) thereof. Meanwhile, a tubular blood inlet port 51 is formed projecting in the lower left region of the heat exchanger housing 5 in Fig. 2. The blood inlet port 51 has a lumen in communication with the blood chamber 50.

In the interior of the heat exchanger housing 5, there are arranged a heat exchange element 54 wholly cylindrical in form, a heating medium chamber-forming member (cylindrical wall) 55 in a cylindrical form provided along the inner periphery of the heat exchange element 54, a partitioning wall 56 separating the inner space of the heating medium chamber-forming member 55 as an inlet heating medium chamber 57 and an outlet heating medium chamber 58. The heating medium chamber-forming member 55 serves to form a heating medium chamber that temporarily stores the heating medium at the inside of the heat exchange element 54 and to regulate the cylindrical heat exchange element from deforming.

The heating medium chamber-forming member 55 and the partitioning wall 56 are fixed in the heat exchanger housing 5 by joining or so due to fusion or an adhesive. The heating medium chamber-forming member 55 and the partitioning wall 56 may be formed as separate members or in one body.

In the heating medium chamber-forming member 55, strip-formed openings 59a, 59b are formed vertically extending and penetrating the wall thereof. The openings 59a, 59b are arranged in opposite positions with respect to the partitioning wall 56 (see Fig. 3). The opening 59a communicates with the inlet heating medium chamber 57 while the opening 59b communicates with the outlet heating medium chamber 58.

The heat exchange element 54 can use a so-called bellows-type heat exchange element (bellows tube) as shown in Fig. 2. The bellows-type heat exchange element 54 has a bellows-formed portion having a multiplicity of hollow annular projections formed nearly parallel in a side surface axially centrally, and cylindrical portions formed at both ends (upper and lower ends) thereof and having a size nearly equal to the inner diameter of the bellows-formed portion. The heat exchange element 54 is formed of a metal material such as stainless steel or aluminum, or a resin material such as polyethylene or polycarbonate, for example. It is preferable to use a metal material, such as stainless steel or aluminum, in respect of strength and heat exchange efficiency. Particularly, it is preferable to structure it by a metal-made bellows tube in a corrugated form having a multiplicity of repetitive concavo-convex portions nearly orthogonal to the axis of the heat exchange element 54.

The heat exchanger housing 5, the heating medium chamber-forming member 55 and the partitioning wall 56 are of a material, e.g. polyolefin such as polyethylene or polypropylene, an ester resin (e.g. polyester such as polyethylene terephthalate or polybutylene terephthalate), a styrene resin (e. g. polystyrene, MS resin or MBS resin), a resin material such as polycarbonate, a ceramics material in various kind or a metal material.

With reference to Figs. 1 to 3, description is now made on the flow of heating medium in the heat exchanging part 1B of the oxygenator 1.

The heating medium, coming through the heating medium inlet port 52, first flows to the inlet heating medium chamber 57 and then to the outer peripheral side of the heating medium chamber-forming member 55 via the opening 59a, thus spreading over the entire periphery of the heating medium-chamber forming member 55 and going into a multiplicity of recesses of the bellows (to the inside of hollow annular projections) of the heat exchange element 54. This heats up or cools down the heat exchange element 54 contacted with the heating medium. Thus, heat exchange (heating or cooling) is effected with the blood flowing along the outer peripheral side of the heat exchange element 54.

The heating medium, served for heating or cooling the heat exchange element 54, enters the outlet heating medium chamber 58 through the opening 59b and then exits at the heating medium outlet port 53.

Note that the heat exchanging part 1B may be not existent in the invention differently from the illustrated embodiment.

With reference to Figs. 1 to 4, description is now made on the blood flow in the oxygenator 1 of this embodiment.

In the oxygenator 1, the blood, coming through the blood inlet port 51, flows into the blood chamber 50, i.e. to between the inner peripheral surface of the heat exchanger housing 5 and the heat exchange element 54, where it goes into contact with the outer surface of the plurality of hollow annular projections of the heat exchange member 54, thus effecting heat exchange (heating or cooling). The blood thus heat exchanged gathers at a downstream portion of the blood chamber 50 and then flows into the housing 2 of the oxygenating part 1A through the blood inlet aperture 24.

The blood, having passed through the blood inlet aperture 24, flows downstream the blood passage 33 formed in the gaps between the hollow fiber membranes 31. Meanwhile, the gas (gas containing oxygen), supplied through the gas inlet port 26, is distributed by the gas inlet chamber 261 into the gas passages 32, i.e. the lumens of the hollow fiber membranes 31. After having passed through the gas passages 32, the gas is collected in the gas outlet chamber 271 and allowed to exit at the gas outlet port 27. The blood, flowing through the blood passage 33, goes into contact with the surfaces of the hollow fiber membranes 31 so that gas exchange (oxygenation, decarboxylation) can be done with the gas flowing through the gas passages 32.

In the case that bubbles present in the gas-exchanged blood, the bubbles are caught by the filter member 4 and are not allowed to flow to the downstream portion of the filter member 4. The bubbles, caught at the filter member 4, once gather at an upstream side of the filter member 4 where they enter the lumens (gas passages 32) of the hollow fiber membranes 31 via a multiplicity of fine pores formed in the wall of the hollow fiber membrane 31 located nearby the same, thus being discharged at the gas outlet port 27 together with the gas flowing the gas passage 32.

The blood, thus subjected to gas exchange and removed of bubbles, flows in the blood outlet aperture 25 where it flows down the blood outlet aperture 25 and exits at the blood outlet port 28.

In the oxygenator 1 of this embodiment, it is preferable to make antithrombotic the surface where to be contacted with blood (e.g. the inner surface of the housing 2, the inner surface of the heat exchanger housing 5, the surface of the heating medium chamber-forming member 55, the surface of the partitioning wall 56, the setting member 7, the surfaces of the partitioning walls 8, 9 facing the blood passage 33) . The antithrombotic surface can be formed by coating and fixing an antithrombotic material over the surface. The antithrombotic material includes heparin, urokinase, HEMA-St-HEMA copolymer, poly-HEMA and so on.

In the oxygenator 1, the flow rate of blood through the blood inlet port 51 is not especially limited because it is different depending upon patient's physique and operation scheme. However, usually, some 0.1 - 2.0 L/min is preferred in infant or child, some 2.0 - 5.0 L/min is preferred in child in elementary or middle school, and some 3.0 - 7.0 L/min is preferred in adult.

In the oxygenator 1, the flow rate of the gas supplied through the gas inlet port 26 is not especially limited because it is different depending upon patient' s physique and operation scheme. However, usually, some 0.05 - 4.0 L/min is preferred in infant or child, some 1.0 - 10.0 L/min is preferred in child in elementary or middle school, and some 1.5 - 14.0 L/min is preferred in adult.

Meanwhile, the oxygen concentration in the gas supplied through the gas inlet port 26 is not especially limited because it is different depending upon the metabolic amount of oxygen/carbon-dioxide gas of a patient under operation. However, it can be taken as 40 - 100%.

Meanwhile, the maximum continuous operation time of the oxygenator 1 is not especially limited because it is different depending upon patient's condition and operation scheme. However, it can be usually taken as approximately 2 - 6 hours. Meanwhile, the maximum continuous operation time of the oxygenator 1 may, rarely, amount to a time as long as nearly 10 hours.

So far, the oxygenator of the invention was described by way of the illustrated embodiment. The invention is not limited to that but, within the scope of the claims, the various elements constituting the oxygenator can be replaced with those in desired structures capable of exhibiting the equivalent function wherein desired elements may be added.

For example, different structures from those illustrated are applicable for the structure or form of the connection between the housing 2 and the heat exchanger housing 5, the forming position and projecting direction of the gas inlet port 26, the gas outlet port 27, the blood outlet port 28, etc. in and from the housing 2, the forming position and projecting direction of the blood inlet port 51, the heating medium inlet port 52 and the heating medium outlet port 53 in and from the heat exchanger housing 5. Meanwhile, the position of the oxygenator 1 in use (positional relationship of various elements relative to the vertical direction) is not limited to those illustrated.

## Claims

1. An oxygenator (1) comprising:
a housing (2) substantially in a rectangular parallelepiped form;
a hollow fiber membrane bundle (3) received in the housing (2) and formed by integrating together a multiplicity of hollow fiber membranes (31) serving for gas exchange, wherein each fiber membrane (31) comprises a lumen structuring a gas passage; and
a gas inlet (26, 261) provided at a gas flow upstream portion of the fiber membrane bundle (3) and a gas outlet (27, 271) provided at a gas flow downstream portion of the fiber membrane bundle (3), so as to provide a gas passage through the lumens of the hollow fiber membranes (31) from the gas flow upstream portion to the gas flow down stream portion; and
a blood-inlet portion (24) extending at a blood flow upstream surface of the fiber membrane bundle (3) and a blood-outlet portion (25) extending at a blood flow downstream surface of the fiber membrane bundle (3), so as to provide a blood passage (33) through the fiber membrane bundle (3) from said blood flow upstream surface to the blood flow downstream surface, wherein the blood passage (33) is formed exterior of the hollow fiber membranes (31);
a filter member (4), serving for catching bubbles, being provided on the blood flow downstream side of the hollow fiber membrane bundle (3);
**characterized in that**
the hollow fiber membrane bundle (3) as a whole has a substantially rectangular parallelepiped form; and **in that**
the filter member (4) is placed such that one of its surfaces is in contact with said blood flow downstream surface of the fiber membrane bundle (3) so that the filter member (4) covers nearly all of the blood flow downstream surface of the fiber membrane bundle (3).

2. An oxygenator (1) according to claim 1 , wherein the filter member (4) is formed by a flat sheet member.

3. An oxygenator (1) according to any preceding claim, wherein the filter member (4) has a rectangular, square, trapezoidal, parallelogram, elliptic or oval form.

4. An oxygenator (1) according to any preceding claim, wherein a gap in the form of a blood outlet aperture (25) is formed between the filter member (4) and the housing (2).

5. An oxygenator (1) according to any preceding claim, wherein the filter member (4) has hydrophilicity.

6. An oxygenator (1) according to any preceding claim, wherein the filter member (4) is in a mesh form.

7. An oxygenator (1) according to claim 6, wherein the filter member (4) has a mesh size of 50 µm or smaller.

8. An oxygenator (1) according to any preceding claim, further comprising a heat exchanging part (1B).

## Patentansprüche

1. Ein Oxygenator (1) mit folgendem:
einem Gehäuse (2), im Wesentlichen in einer Form eines rechtwinkligen Parallelepipedon;
einem Bündel (3) von Hohlmembranfasern, aufgenommen in dem Gehäuse (2) und **dadurch** ausgebildet, dass eine Vielzahl von zum Gasaustausch dienenden Hohlmembranfasern (31) zusammen integriert ist, wobei jede Membranfaser (31) ein einen Gasdurchlass bildendes Lumen umfasst;
einem Gaseinlass (26, 261), bereitgestellt an einem bezüglich eines Gasflusses stromaufwärtigen Bereich des Membranfaserbündels (3), und einem Gaseinlass (27, 271), bereitgestellt an einem bezüglich des Gasflusses stromabwärtigen Bereich des Membranfaserbündels (3), so dass ein Gasdurchlass durch die Lumen der Hohlmembranfasern (31) von dem bezüglich des Gasflusses stromaufwärtigen Bereichs zu dem bezüglich des Gasflusses stromabwärtigen Bereichs bereitgestellt wird;
einem Bluteinlassbereich (24), der sich an einer bezüglich eines Blutflusses stromaufwärtigen Oberfläche des Membranfaserbündels (3) erstreckt, und ein Blutauslassbereich (25), der sich an einer bezüglich des Blutflusses stromabwärtigen Oberfläche des Membranfaserbündels (3) erstreckt, so dass ein Blutdurchlass (33) durch das Membranfaserbündel (3) von der bezüglich des Blutflusses stromaufwärtigen Oberfläche zu der bezüglich des Blutflusses stromabwärtigen Oberfläche bereitgestellt wird, wobei der Blutdurchlass (33) außerhalb der Hohlmembranfasern (31) ausgebildet ist; und
einem Filterelement (4), das zum Abfangen von Blasen dient und das auf der bezüglich des Blutflusses stromabwärtigen Seite des Hohlmembranfaserbündels (3) angeordnet ist;
**dadurch gekennzeichnet, dass**
das Hohlmembranfaserbündel (3) als ein Ganzes eine im wesentlichen rechtwinklige Parallelepipedonform aufweist; und **dadurch** dass
das Filterelement (4) so angeordnet ist, dass eine seiner Oberflächen in Kontakt mit der bezüglich des Blutflusses stromabwärtigen Oberfläche des Membranfaserbündels (3) ist, so dass das Filterelement (4) nahezu die gesamte bezüglich des Blutflusses stromabwärtige Oberfläche des Membranfaserbündels (3) bedeckt.

2. Ein Oxygenator (1) nach Anspruch 1, wobei das Filterelement (4) als ein flaches Plattenelement ausgebildet ist.

3. Ein Oxygenator (1) nach einem der vorhergehenden Ansprüche, wobei das Filterelement (4) eine rechtwinklige, quadratische, trapezförmige parallelogrammförmige, elliptische oder ovale Form aufweist.

4. Ein Oxygenator (1) nach einem der vorhergehenden Ansprüche, wobei ein Zwischenraum in einer Form einer Blutauslassöffnung (25) zwischen dem Filterelement (4) und dem Gehäuse (2) ausgebildet ist.

5. Ein Oxygenator (1) nach einem der vorhergehenden Ansprüche, wobei das Filterelement (4) hydrophile Eigenschaften aufweist.

6. Ein Oxygenator (1) nach einem der vorhergehenden Ansprüche, wobei das Filterelement (4) in einer Netz- bzw. Gewebeform (ausgebildet) ist.

7. Ein Oxygenator (1) nach Anspruch 6, wobei das Filterelement (4) eine Maschenweite von 50 µm oder kleiner aufweist.

8. Ein Oxygenator (1) nach einem der vorhergehenden Ansprüche, der ferner eine Wärmeaustauschbaugruppe (1B) umfasst.

## Revendications

1. Oxygénateur (1) comprenant :
un boîtier (2) substantiellement de forme parallélépipédique rectangulaire ;
un faisceau de membranes de fibres creuses (3) logé dans le boîtier (2) et formé en intégrant ensemble une multitude de membranes de fibres creuses (31) servant pour l'échange gazeux, dans lequel chaque membrane de fibres (31) comprend une lumière structurant un passage de gaz ; et
une entrée de gaz (26, 261) placée dans une partie amont d'écoulement de gaz du faisceau de membranes de fibres (3) et une sortie de gaz (27, 271) placée dans une partie aval d'écoulement de gaz du faisceau de membranes de fibres (3), afin de fournir un passage de gaz à travers les lumières des membranes de fibres creuses (31), de la partie amont d'écoulement de gaz vers la partie aval d'écoulement de gaz ; et
une partie d'entrée de sang (24) s'étendant au niveau d'une surface amont d'écoulement sanguin du faisceau de membranes de fibres (3) et une partie de sortie de sang (25) s'étendant au niveau d'une surface aval d'écoulement sanguin du faisceau de membranes de fibres (3), afin de fournir un passage sanguin (33) à travers le faisceau de membranes de fibres (3) de ladite surface amont d'écoulement sanguin vers la surface aval d'écoulement sanguin, dans lequel la passage sanguin (33) est formé à l'extérieur des membranes de fibres creuses (31) ;
un élément filtrant (4), servant à piéger les bulles, étant prévu du côté aval de l'écoulement sanguin du faisceau de membranes de fibres (3) ;
**caractérisé en ce que** :
le faisceau de membranes de fibres (3) dans son ensemble a une forme parallélépipédique substantiellement rectangulaire ; et **en ce que**
l'élément filtrant (4) est placé de telle manière que l'une de ses surfaces est en contact avec ladite surface aval d'écoulement sanguin du faisceau de membranes de fibres (3) de sorte que l'élément filtrant (4) couvre presque toute la surface aval d'écoulement sanguin du faisceau de membranes de fibres (3).

2. Oxygénateur (1) selon la revendication 1, dans lequel l'élément filtrant (4) est formé par un élément en feuille plate.

3. Oxygénateur (1) selon l'une quelconque des revendications précédentes, dans lequel l'élément filtrant (4) a une forme rectangulaire, carrée, trapézoïdale, en parallélogramme, elliptique ou ovale.

4. Oxygénateur (1) selon l'une quelconque des revendications précédentes, dans lequel un espace sous la forme d'une ouverture de sortie de sang (25) est formée entre l'élément filtrant (4) et le boîtier (2).

5. Oxygénateur (1) selon l'une quelconque des revendications précédentes, dans lequel l'élément filtrant (4) a un caractère hydrophile.

6. Oxygénateur (1) selon l'une quelconque des revendications précédentes, dans lequel l'élément filtrant (4) se présente sous la forme d'un tamis.

7. Oxygénateur (1) selon la revendication 6, dans lequel l'élément filtrant (4) a une taille de maille de 50 µm ou moins.

8. Oxygénateur (1) selon l'une quelconque des revendications précédentes, comprenant en outre une partie d'échange de chaleur (1B).
